# EUROPEAN PATENT SPECIFICATION

(11) **EP 1 658 063 B1**
(45) Date of publication and mention of the grant of the patent: **12.03.2014**
(21) Application number: 03817895.0
(22) Date of filing: 06.08.2003
(51) Int. Cl.: A61K 31/195, A61K 31/167, A61K 31/216, A61K 31/138, A61K 9/14, A61P 11/00, A61P 11/08

(54) **ADVANTAGEOUS COMBINATIONS FOR INHALATION OF NACYSTELYN AND BRONCHODILATORS**
VORTEILHAFTE MISCHUNGEN VON NACYSTELYN UND BRONCHODILATATOREN ZUR INHALATION
COMBINAISONS AVANTAGEUSES POUR INHALATION DE NACYSTELYN ET DE BRONCHODILATATEURS

(43) Date of publication of application: 24.05.2006
(73) Proprietor: Galephar M/F, 6900 Marche-en-Famenne (BE)
(72) Inventor: VANDERBIST, Francis, B-1650 Beersel (BE); BAUDIER, Philippe, B-1180 Uccle (BE); DEBOECK, Arthur, Puerto Rico 00778 (US)
(74) Representative: Decamps, Alain René François
(86) International application number: PCT/BE2003/000134
(87) International publication number: WO 2005/013963

(56) References cited:
- EP-A- 0 876 814
- WO-A-98/48839
- WO-A-03/035137
- US-A- 4 847 282
- ZA-A- 9 400 155
- WORTH H. ET AL: "Leitlinie der Deutschen Atemwegsliga und der Deutschen Gesellschaft für Pneumologie zur Diagnostik und Therapie von Patienten mit chronisch obstruktiver Bronchitis und Lungenemphysem (COPD)", PNEUMOLOGIE, vol. 56, no. 11, 20 November 2002 (2002-11-20), pages 704-738,
- ANONYMOUS: "Preparations for Inhalation: Aerodynamic Assessment of Fine PArticles", EUROPEAN PHARMACOPOEIA,, 1 April 2005 (2005-04-01), pages 3103-3115, XP007921794,

## Description

### BACKGROUND OF THE INVENTION

The main chronic respiratory diseases are Chronic Obstructive Pulmonary Disease (COPD), asthma and Cystic Fibrosis (CF).

The definition of COPD originating from the Merck Manual of Diagnosis and Therapy, published by Merck Research Laboratories, Division of Merck & Co., Inc., Whitehouse Station, N.J., seventeen edition, 1999 is the following:

A disease characterized by chronic bronchitis or emphysema and airflow obstruction that is generally progressive, may be accompanied by airway hyperreactivity, and may be partially reversible.

On the other hand, asthma is characterized by airway inflammation that is manifested by airway hyperresponsiveness to a variety of stimuli and by airway obstruction that is reversible spontaneously or in response to treatment; reversibility may be incomplete in some patients.

Cystic fibrosis is another chronic inflammatory disease defined as follows: An inherited disease of the exocrine glands, primarily affecting the GI and respiratory systems, and usually characterized by COPD, exocrine pancreatic insufficiency, 30 and abnormally high sweat electrolytes.

The various terms used to quantify the pulmonary functions are described hereinbelow (Quanjer and al., Eur. Respir. J., 1993, 6, Suppl. 16, 5-40, Official Statement of the European Respiratory Society).

Forced Vital Capacity (FVC): is the volume of gas delivered during an expiration made as forcefully and completely as possible starting from full inspiration.

Forced expiratory volume in one second (FEV1): is the volume of gas exhaled in a one second from the start of the forced vital capacity manoeuvre.

Peak Expiratory Flow Rate (PEFR): is the maximal flow during a forced expiratory vital capacity manoeuvre starting from a position of full inspiration.

Chronic obstructive pulmonary disease (COPD), which includes chronic bronchitis and emphysema, is steadily increasing in frequency, possibly due to continued smoking, increasing air pollution, and the continued ageing of the population.

Prevalence: in the Global Burden of Disease Study conducted under the auspices of the WHO and the World Bank. The world-wide prevalence of COPD in 1990 was estimated to be 9.34/1,000 in men and 7.33/1,000 in women. However, these estimates include all ages and underestimate the true prevalence of COPD in adults. The prevalence of COPD is highest in countries where cigarette smoking has been, or still is, very common, while the prevalence is lowest in countries where smoking is less common, or total tobacco consumption per individual is low.

Morbidity: the limited data that are available indicate that morbidity due to COPD increases with age and is greater in men than women; COPD is responsible for a significant part of physician visits, emergency department visits and hospitalizations.

Mortality: COPD is currently the fourth leading cause of death in the world, and further increases in the prevalence and mortality of the disease can be predicted in the coming decades. In the US, COPD death rates are very low among people under age 45 but then increase with age, and COPD becomes the fourth or fifth leading cause of death among those over 45.

COPD is characterized by edema (oedema) of the mucous membrane, which lines the interior walls of the tracheobronchial tree. When the mucosa accumulates an abnormal quantity of liquid, the profuse and thickened serous fluid excreted may seriously affect ventilation in the alveoli. The mucus resists movement up the walls of the tracheobronchial tree, normally efficiently accomplished by the cilia throughout the airways. Consequently, the serous fluid can form mucus plugs, which can shut off alveoli or an entire airway depriving whole sections of the lung of oxygen-rich air.

Plugs of mucus in the tracheobronchial tree may only partially block the flow of air through the bronchioles. This partial blockage can create a turbulent flow of air, which forms bubbles on the surface of mucosa. When there are enough bubbles, they become foam, which can clog airways and dramatically diminish respiration of the capillaries of the lungs.

The obstruction of the bronchi and bronchioles found in COPD is often a severely disabling condition. A wide variety of compounds including oral methylxanthines, oral and inhaled beta-adrenergic agonists, inhaled cromolyn sodium, inhaled anticholinergics, and oral and inhaled corticosteroids have been tested. Despite the existence of these therapeutic tools, a large number of patients are not responsive to these medications or become non-responsive after a prolonged period of treatment. COPD is always accompanied by an important oxidative stress resulting from an oxidant/antioxidant imbalance, an excess of oxidants and/or a depletion of antioxidants. Oxidative stress is thought to play an important role in the pathogenesis of a number of lung diseases, not only through direct injurious effects, but by involvement in the molecular mechanisms that control lung inflammation. A number of studies have shown an increased oxidant burden and consequently increased markers of oxidative stress in the airspaces, breath, blood, and urine in smokers and in patients with COPD. The presence of oxidative stress has important consequences for the pathogenesis of COPD. These include oxidative inactivation of antiproteinases, airspace epithelial injury, increased sequestration of neutrophils in the pulmonary microvasculature, and gene expression of proinflammatory mediators. With regard to the latter, oxidative stress has a role in enhancing the inflammation that occurs in smokers and patients with COPD, through the activation of redox-serisitive transcription factors such as nuclear factor-κB and activator protein-1, which regulate the genes for proinflammatory mediators and protective antioxidant gene expression.

Asthma is the most common form of bronchoconstrictive disease, which is completely different from COPD. Pathologically, asthma involves constriction of the bronchioles, hypertrophy of the muscles of the bronchioles, and a characteristic infiltrate of eosinophils.

Asthma is the third leading cause of preventable hospitalization in United States. There are about 470.000 hospitalizations and more than 5.000 deaths a year from asthma. Asthma causes recurring episodes of coughing, wheezing, chest tightness, and difficult breathing. Asthma attacks can be life threatening. They can be prevented. Asthma is a chronic inflammatory disorder of the airways. Chronically inflamed airways are hyperresponsive; they become obstructed and airflow is limited (by bronchoconstriction, mucus plugs, and increased inflammation) when airways are exposed to various stimuli, or triggers.

Common asthma triggers (that is, factors that make asthma worse) include viral infections; allergens such as domestic dust mites (in bedding, carpets, and fabricupholstered furnishings), animals with fur, cockroach, pollens, and molds; tobacco smoke; air pollution, exercise; strong emotional expressions; chemical irritants, and drugs (aspirin and beta blockers).

Asthma attacks (or exacerbations) are episodic, but airway inflammation is chronically present. Asthma is a chronic disorder requiring long-tern management. For many patients, this means taking preventive medication every day.

Asthma can change over time. Asthma can be mild, moderate or severe; asthma attacks can be life-threatening. The severity of asthma varies among individuals, and it can change in one individual over time. Treatment decisions are made based on the severity of asthma.

In conclusion, it is clear that a chronic inflammatory diseases of the lungs like COPD and asthma present simultaneously important inflammation and / or oxidation phenomenons of the lung tissue and a significant bronchoconstriction, making the breathing of patients very difficult. No efficient pharmaceutical treatment is currently available to treat all those symptoms simultaneously.

A number of patents relating to the treatment of COPD or other pathologies of the respiratory tract have already been granted.

The US Patent 6,153,187 describes a method of managing a patient having an accumulation of mucoid, mucopurulent or purulent material containing glycosaminoglycans, the method comprising the step of administering at least one glycosaminoglycan degrading enzyme to the patient in an amount therapeutically effective to reduce at least one of the following: the visco-elasticity of the material, pathogens infectivity and inflammation.

The US Patent 5,969,421 describes a method for prevention of cell death, and method comprising simultaneously topically applying ACC and levulose as an application in an amount effective to result in a synergistic action protecting cells, and presenting cell damage caused by various agents. A pharmaceutical preparation including a combination of ACC and levulose.

The WO Patent 9635452 describes a pharmaceutical composition useful in the treatment of respiratory tract disorders. The composition comprises as active ingredients: (a) acetylcysteine, carbocysteine, erdocysteine or a pharmaceutically acceptable salt of any of these; and (b) a beta 2 agonist, e.g. salbutamol, terbutaline; and (c) an expectorant e.g. guaiphenesin, sodium citrate; ammonium chloride.

The WO Patent 0010598 describes a method of treating mucus hypersecretion, the causative factor in COPD, asthma and other clinical conditions involving COPD, comprises administering a compound that inhibits exocytosis in mucus secreting cells or neurons that control or direct mucus secretion.

Some inventions describe the combination of a β2 mimetics with an inhaled corticosteroid for the treatment of asthma and COPD. Examples of such inventions are EP416950, which describes the combination of salmeterol with beclomethasone; EP416951 relating to the combination of salmeterol with fluticasone or US 5,674,860 which relates to the combinations of formoterol and budesonide.

L-Lysine N-Acetylcysteinate (also called Nacystelyn or NAL) (US patent 4,847,282) is an active ingredient efficient in the treatment of chronic inflammatory diseases such as, but not limited to cystic fibrosis (CF) and chronic obstructive pulmonary disease (COPD) (Am. J. Respir. 2000, 161(3) - A72, pediatric pulmonology 22, 161-166 (1996), Free Rad. Biol. Med. 2002, 32(6), 492-502). NAL is actually a water-soluble salt of acetylcysteine (ACC), a widely used mucolytic. A number of experiments have demonstrated that NAL presents several advantages over ACC. The most important of them being the almost neutral pH of NAL (pH ± 6.5) what makes its administration possible via the inhalation route without observing any bronchospasms, contrary to ACC, which presents an acidic pH (pH ± 2.2) that can be responsible for reflex bronchospasms. This property allows the safe administration of NAL to patients by inhalation via all the inhaled technologies i.e.: nebulization, metered dose inhalers (MDIs) or drug powder inhalers, without the need of any buffering agents (Eur. Respir. J., 1994, 7(1), 8187.

L-lysine N-acetylcysteinate (CAS N°89344-48-9) has been patented several years ago (US Patent 4,847,282, "Mucolytic acetylcysteine salts"). The principle of the invention was the synthesis of a water soluble acetylcysteine salt, consisting of reaction products of acetylcysteine with at least one basic amino-acid, the latter being preferably selected from the group comprising arginine, lysine, histidine, ornithine and glycine. A number of experiments have been performed to demonstrate that NAL presents several advantages over ACC, its parent molecule: the most important advantage is that NAL has an almost neutral pH (pH = 6.5), what makes its administration possible via the inhalation route without observing any side effects (bronchospasms), contrary to ACC which is acidic (pH 2.2). This allows the safe administration of NAL by inhalation by every galenical form available for this route e.g. nebulization, Metered Dose Inhalers (MDI) and Dry Powder Inhalers (DPI).

EP-A-0876 814 describes the use of L-Lysine-N-acetylcysteinate (NAL) as mucolytic agent alone in a dry powder inhaler (DPI) for the treatment of cystic fibrosis or chronic obstructive respiratory diseases.

A number of experiments have been performed to assess the mucolytic, antioxidant and antiinflammatory properties of NAL in comparison to ACC. It is also important to mention that NAL is active at lower doses than ACC what makes its administration possible by inhalation via portable inhaler devices (DPI or MDI device).

This also greatly facilitates the administration for the patient and hence the compliance since there is no need to use a cumbersome nebulizer which requires an administration time of 10 to 30 minutes, what is difficult to accept for ambulatory patients like the majority of COPD or asthma patients. When NAL is formulated as a dry powder inhaler using a capsule device, the mean administration time for the patient is between 30 and 60 seconds.

The possibility of using a very convenient device and allowing to reach a high lung deposition of NAL is also very advantageous for the treatment of an ambulatory disease like COPD and asthma.

In the past, it has been demonstrated that surprisingly NAL presents a mucolytic and antioxidant activity superior to the activity of ACC (Vanderbilt and al Arzneim-Forschung/ Drug Research (II) N°8, 1996, Nagy and al., Pulmonary Pharmacology 2 Therapeutics (1997) 10, 287-292, Gillissen and al Respiratory Medicine (1997), 91, 159-168, Tomkiewicz and al. Pulmonary Pharmacology (1995) 8, 259-265, Marriott and al, Eur. Resp. J., 6 (suppl. 17), 438 (5) (abstract), 1993.

For some properties (mucolytic), the superiority of NAL may be explained while for other (antioxidant and antiinflammatory) effects, we can only make some hypothesis about the potential mode of action of the molecule.

Those mucolytic, antioxidant and antiinflammatory properties of NAL result in significant clinical improvement in patients. For instance, it has been demonstrated in cystic fibrosis patients that the administration of NAL can significantly decrease the number and severity of lung exacerbations in comparison to placebo.

Bronchodilators are a very widely used treatment of chronic respiratory diseases. Although they can act through various mechanisms of action, they all result in the dilatation of the airways of the respiratory tract, resulting in an improvement of patient's lung functions such as Forced Vital Capacity (FVC), Forced Expiratory Volume in one second (FEV1) and Peak Expiratory Flow Rate (PEFR). There are two main classes of bronchodilators: the sympaticomimetics, including the short acting and the long-acting β2-mimetics, and the anticholinergics.

Short-action β2-mimetics include salbutamol, terbutaline, fenoterol, pirbuterol or tulobuterol. Each active ingredient cited can be used as the base or as an acceptable pharmaceutical salt. The long-acting β2 mimetics include formoterol and salmeterol. Each molecule can be used as base or as an acceptable pharmaceutical salt. The anticholinergic drugs include tiotropium.

Worth H. et al. (Pneumologie, vol.56, no. 11, 20 November, 2002, pages 704-738) discloses the use of bronchodilators with long duration alone, e.g. salmoterol, formoterol and tiotropium for the treatment of chronic obstructive pulmonary diseases.

In general, bronchodilators are used in asthma and chronic obstructive pulmonary diseases but can be administered in almost every inflammatory disease of the lung.

ZA-A-9400155 discloses the combination of N-acetylcystein (ACC) with a short acting bronchodilator for enhancing the clearance of bronchial secretion via the dual action of bronchodilation and lowering the viscosity of mucous secretions. However, the efficacy of the dual composition of said application is not effective enough to treat COPD, therefore it is highly desirable to obtain the compositions enhancing the efficacy of said compositions, in particular, to increase the penetration depth of DPI composition for treating COPD and asthma while preventing an acute attack which can endanger a COPD patient's life.

### FIELD OF THE INVENTION

Chronic respiratory diseases involve complexes mechanisms and require the administration of different kinds of treatment to patients among with antibiotics, corticosteroids, bronchodilators and mucolytics. The field of the present invention is to provide an advantageous, efficient and safe treatment of such diseases by administering a dry powder pharmaceutical composition as defined herein. Advantageous combinations are disclosed in the attached claims.

The present invention is defined in the appended independent claims. Preferred embodiments are defined in the dependent claims.

It has now surprisingly been found that NAL and bronchodilator agent with very long duration of action which has to be administered only once a day which is the anticholinergic drug tiotropium or a long acting β2-mimetic can advantageously be combined to be used by inhalation in the treatment of chronic respiratory diseases such as cystic fibrosis, chronic obstructive pulmonary disease or asthma. By "anticholinergic drug with a long duration of action" is meant drugs which need be administered only once a day; a long acting β2-mimetic usually is to be administered twice a day.

NAL has a duration of action that makes its administration possible from 1 to 4 times a day. The bronchodilator class of molecules suitable for the present invention includes molecules with very long duration of action which have to be administered only once a day (tiotropium) but also long acting β2-mimetics which are usually administered twice a day like formoterol and salmeterol. Consequently, NAL can be combined in a single pharmaceutical form with the long duration bronchodilators as defined in the claims. NAL and long duration bronchodilators as defined in the claims offer complementary modes of action which make their administration particularly profitable for the patients. Indeed, NAL, through its mucolytic activity may allow to the bronchodilator to be deposited more deeply in the lungs after inhalation i.e. in its target tissue, the bronchi and the bronchioli, so permitting a better efficacy of the bronchodilator agent. On the other hand, through its action of dilatation of the conducting airways, the bronchodilator agent will allow to NAL to also be deposited deeply in the lungs thus guarantying an optimal antiinflammatory and antioxydant action of NAL, all along the respiratory tract.

Using such combination therapy, medicaments which result in a significant improvement in lung function may be prepared. In another aspect, using the combination therapy of the invention, medicaments which provide improved control of obstructive or inflammatory airways diseases, or a reduction in exacerbation's of such diseases, may be prepared. In a further aspect, using compositions of the invention, medicaments which can be used on demand in rescue treatment of obstructive or inflammatory airways diseases, or which reduce or eliminate the need for treatment with short-acting rescue medicaments such as salbutamol or terbutaline, may be prepared; thus medicaments based on compositions of the invention facilitate the treatment of an obstructive or inflammatory airways disease with a single medicament.

The combination allowing an administration twice or once a day will be preferred to combinations requiring more administrations because they increase the comfort and the compliance of the patients. NAL and the bronchodilator can be administered simultaneously, separately or sequentially. The combination of NAL and the bronchodilator agent can be formulated as a drug powder inhaler (DPI).

For a more easier understanding of which kind of combination the present invention is relating, in the following of the present text, the letter (A) will be used to design Nacystelyn and the letter (B) will be used to design the bronchodilator agent which is (i) tiotropium or (ii) a long acting β2-mimetic. The present invention consists in the administration of the medicament or pharmaceutical composition as hereinbefore described, i.e. with (A) and (B) in admixture by inhalation, i.e. the mixture of (A) and (B) are in inhalable form.

The inhalable form is a dry powder, i.e. (A) and (B) are present in a dry powder comprising finely divided (A) and (B) optionally together -with a finely divided pharmaceutically acceptable carrier, which is preferably present and may be one or more materials known as pharmaceutically acceptable carriers, preferably chosen from materials known as carriers in dry powder inhalation compositions, for example saccharides, including monosaccharides, disaccharides, polysaccharides and sugar alcohols such as arabinose, glucose, fructose, ribose, mannose, sucrose, trehalose, lactose, maltose, starches, dextran or mannitol. An especially preferred carrier is lactose. The dry powder may be in capsules of hard gelatin or hydroxypropylmethylcellulose, or in blisters, for use in a dry powder inhalation device, preferably in dosage units of (A) and (B) together with the carrier in amounts to bring the total weight of powder per dose to from 5 mg to 50 mg. Alternatively, the dry powder may be contained as a reservoir in a multi-dose dry powder inhalation device.

In the finely divided particulate form of the medicament, and in the aerosol composition where the active ingredient is present in particulate form, the active ingredient may have an average particle diameter of up to about 10 µm, for example 0.1 to 5 µm, preferably 1 to 5 µm. The solid carrier, where present, generally has a maximum particle diameter up to 300 µm, preferably up to 212 µm, and conveniently has a mean particle diameter of 100 to 160 µm, e.g. 100 to 125 µm. The particle size of the active ingredient, and that of a solid carrier where present in dry powder compositions, can be reduced to the desired level by conventional methods, for example by grinding in an air-jet mill, ball mill or vibrator mill, microprecipitation, spray-drying, lyophilisation or recrystallization from supercritical media, the carrier material can also consist in a mix of different materials in order to optimize the properties of the dry powder inhaler formulation. The inhalable medicament may be administered using an inhalation device suitable for the inhalable form, such devices being well known in the art. Accordingly, the invention also provides a pharmaceutical product comprising a medicament or pharmaceutical composition as hereinbefore described in inhalable form as hereinbefore described in association with one or more inhalation devices. In a further aspect, the invention discloses an inhalation device, or a pack of two or more inhalation devices, containing a medicament or pharmaceutical composition as hereinbefore described in inhalable form as hereinbefore described.

### Examples:

The invention is additionally illustrated in connection with the following examples, which are considered to be illustrative of the present invention. It should be understood, however, that the invention is not limited to the specific details of the Examples.

### Comparative Example 1: NAL / salmeterol 3 / 0.050 mg / puff

The salmeterol used was under the form of salmeterol xinafoate. 73.2 mg of salmeterol xinafoate are equivalent to 50.0 mg of salmeterol base.

### Formula:

| ingredient | Amount / puff (mg / puff) |
|---|---|
| Nacystelyn | 4 |
| salmeterol xinafoate | 0.073 |
| lecithin | 7.5 |
| monofluorotrichloroethane | 20.0 |
| difluorodichloromethan | 50.0 |

### Example 2: combination NAL / Formoterol - dry powder inhaler formulation

### Formula:

| ingredient | Amount / dose(mg / dose) |
|---|---|
| Nacystelyn | 4.0 |
| Formoterol fumarate | 0.006 |
| Lactose monohydrate | 5.90 |

Process: formoterol fumarate (with a weight average particle size comprised between 1 and 5 µm) is pre-blended with a small fraction of lactose (1/10 of the total amount of lactose with a weight particle size comprised between 100 and 300µm, such as about 160µm,) in a cubic mixer (Turbula). NAL (with a weight average particle size comprised between 1 and 5 µm) is blended in a planetary mixer (Colette) with the remaining part of lactose (9/10 of the amount of lactose) for 10 minutes on speed 1. The pre-blend formoterol / lactose is then added to the NAL / lactose mix in the planetary mixer (sandwich mix) and the final blend is mix at speed 1 for another 10 minutes. The resulting powder is then manually filled into Multidose DPI device (Mulidose MIAT Inhaler, Milano, Italy). Another part of the mix has been filled into size 3 hydroxypropylmethylcellulose capsules to be used with a single dose DPI device (Miat Monodose inhaler).

### Example 3: combination NAL / Formoterol fumarate - dry powder inhaler formulation 10 / 0.012 mg

### Formula:

| ingredient | Amount / capsule (mg / capsule) |
|---|---|
| Nacystelyn | 10.0 |
| Formoterol fumarate | 0.012 |
| Lactose monohydrate | 8.0 |
| Anhydrous lactose | 22.0 |

The mixing process was the same as in example 2.

The powder was filled into transparent - transparent, size 3 hydroxypropylmethylcellulose capsules and adminsitered with the monodose Miat inhaler (Miat, Milano, Italy).

### Example 4: Stability data on NAL / formoterol DPI from example 3

Stability data on NAL / formoterol capsules packaged in aluminum / aluminum blisters.

### A)25°C/60%RH

| | | T0 | T3 Months | T6 Months | T12 Months | T18 Months | T24 Months |
|---|---|---|---|---|---|---|---|
| ***Assay*** | | | | | | | |
| | formoterol (%) | 99.8 | 98.9 | 99.6 | 99.2 | 99.1 | 99.3 |
| | NAL (%) | 101.2 | 100.6 | 100.4 | 100.7 | 100.8 | 100.2 |

| ***Impurities*** | | | | | | | |
|---|---|---|---|---|---|---|---|
| | **a) formoterol** | | | | | | |
| | impurity 52008RC01 (%) | 0.2 | 0.3 | 0.2 | 0.3 | 0.4 | 0.5 |
| | total impurities (%) | 0.2 | 0.3 | 0.3 | 0.4 | 0.5 | 0.6 |
| | **b) NAL** | | | | | | |
| | NN-diaceylcystine (%) | 0.15 | 0.20 | 0.22 | 0.30 | 0.32 | 0.46 |
| | total impurites (%) | 0.20 | 0.22 | 0.25 | 0.26 | 0.27 | 0.28 |

| ***Fine particle Dose*** | | | | | | | |
|---|---|---|---|---|---|---|---|
| | formoterol (µg) | 3.7 | 3.6 | 3.8 | 3.6 | 3.9 | 3.8 |
| | NAL (mg) | 3.2 | 3.1 | 3.1 | 3.0 | 3.2 | 3.1 |

The stability tests were carried out in order to establish the presence of impurities and the fine particle dose after preparation, as well as after a storage period of 3 months, 6 months, 12 months, 18 months and 24 months at 25°C with a relative humidity of 60%. This test shows the stability of the composition with respect to the formation of impurities, as well as with respect to the fine particle dose for the formoterol and for the NAL.

### Example 5: combination NAL / Salmeterol - dry powder inhaler formulation 10/0.073 mg (equal to 50. 0 mg of salmeterol base)

| ingredient | Amount / capsule (mg / capsule) |
|---|---|
| Nacystelyn | 10.0 |
| Salmeterol xinafoate | 0.073 |
| Lactose monohydrate | 8.0 |
| Anhydrous lactose | 22.0 |

### Example 6: Fine particle Dose (FPD) obtained with the DPI combination NAL / Salmeterol of example 5 versus the marketed DPI form of salmeterol DPI (Serevent 50 µg, Diskus)

From an in vitro comparative deposition of salmeterol from (i) the combination NAL / salmeterol 10 / 0.025 mg of the current invention versus (ii) the Serevent 50 µg, Diskus ( Deposition measured with a Multistage Liquid Impinger, 4 liters of air, n=3), it appears that the fine particle dose obtained with the combination of the invention (NAL + 25µg salmetertol) was 10.9 µg salmeterol, while said fine particle dose was 9.89 µg, salmeterol for the Serevent 50µg salmeterol

## Claims

1. A dry powder pharmaceutical composition containing a combination of:
a) a L-lysine N-acetylcysteinate (NAL); and
b) a bronchodilator agent which is:
i, tiotropium; or
ii. a long acting β2-mimetic,
for use in treating inflammatory or obstructive respiratory disease wherein the composition is administered by inhalation.

2. Composition for use according to claim 1, wherein the long acting β2-mimetic is Formoterol or a solvate thereof, or salmeterol or salts thereof, or mixtures thereof.

3. Composition for use according to any of the preceding claims, adapted for an administration twice or once a day.

4. Composition for use according to any of the preceding claims, wherein the composition contains in addition of (a) and (b) at least one pharmaceutically acceptable carrier.

5. Composition for use according to claim 4, wherein the carrier has a maximum particle diameter up to 300 µm.

6. Composition for use according to claim 4 or 5, wherein the carrier is a saccharide.

7. Composition for use according to claim 6, wherein the saccharide is a monasaccharide, disaccharide, polysaccharide or sugar alcohols.

8. Composition for use according to claim 7, wherein the saccharide is arabinose, glucose, fructose, ribose, mannose, sucrose, trehalose, lactose maltose, starches, dextran or mannitol.

9. Composition for use according to claim 8, wherein the saccharide is lactose.

10. Composition for use according to any of the preceding claims, wherein the composition is in a form suitable for inhalation through a monodose inhaler device.

11. Composition for use according to any of the preceding claims, wherein said composition being in a form suitable for inhalation through a multiple-dose inhaler.

12. Composition for use according to any one of claims 1 to 11 **characterized in that** it is contained in a capsule of hard gelatin or hydroxypropylmethylcellulose.

13. Composition for use according to any one of claims I to 11 **characterized in that** it is contained in a blister,

## Patentansprüche

1. Pharmazeutische Trockenpulverzusammensetzung, umfassend eine Kombination aus:
a) einem L-Lysin-N-Acetylcysteinat (NAL); und
b) einem bronchienerweiternden Mittel, das Folgendes darstellt:
i. Tiotropium; oder
ii. ein langzeitwirkendes β2-Mimetikum,
zur Verwendung bei der Behandlung einer entzündlichen oder obstruktiven Atemwegserkrankung, wobei die Zusammensetzung durch Inhalation verabreicht wird.

2. Zusammensetzung zur Verwendung nach Anspruch 1, wobei das langzeitwirkende β2-Mimetikum Formoterol oder ein Solvat daraus oder Salmeterol oder Salze daraus oder Mischungen daraus ist.

3. Zusammensetzung zur Verwendung nach einem der vorhergehenden Ansprüche, die für eine zweimalige oder einmalige Verabreichung pro Tag ausgelegt ist.

4. Zusammensetzung zur Verwendung nach einem der vorhergehenden Ansprüche, wobei die Zusammensetzung zusätzlich zu (a) und (b) mindestens eine pharmazeutisch akzeptable Trägersubstanz umfasst.

5. Zusammensetzung zur Verwendung nach Anspruch 4, wobei die Trägersubstanz einen maximalen Partikeldurchmesser von bis zu 300 µm aufweist.

6. Zusammensetzung zur Verwendung nach Anspruch 4 oder 5, wobei die Trägersubstanz ein Saccharid ist.

7. Zusammensetzung zur Verwendung nach Anspruch 6, wobei das Saccharid ein Monosaccharid, ein Disaccharid, ein Polysaccharid oder Zuckeralkohole ist.

8. Zusammensetzung zur Verwendung nach Anspruch 7, wobei das Saccharid Arabinose, Glukose, Fruktose, Ribose, Mannose, Saccharose, Trehalose, Laktose, Maltose, Stärken, Dextran oder Mannitol ist.

9. Zusammensetzung zur Verwendung nach Anspruch 8, wobei das Saccharid Laktose ist.

10. Zusammensetzung zur Verwendung nach einem der vorhergehenden Ansprüche, wobei die Zusammensetzung eine Form aufweist, die zur Inhalation durch eine Monodosis-Inhalationsvorrichtung geeignet ist.

11. Zusammensetzung zur Verwendung nach einem der vorhergehenden Ansprüche, wobei die Zusammensetzung eine Form aufweist, die zur Inhalation durch eine Multidosis-Inhalationsvorrichtung geeignet ist.

12. Zusammensetzung nach einem der Ansprüche 1 bis 11, **dadurch gekennzeichnet, dass** sie in einer Kapsel aus Hartgelatine oder aus Hydroxypropylmethylcellulose enthalten ist.

13. Zusammensetzung nach einem der Ansprüche 1 bis 11, **dadurch gekennzeichnet, dass** sie in einem Blister enthalten ist.

## Revendications

1. Composition pharmaceutique de poudre sèche contenant une combinaison de :
a) un N-acétylcystéinate de L-lysine (NAL) ;
et
b) un agent bronchodilatateur qui est :
i. le tiotropium ; ou
ii. un β2-mimétique à longue durée d'action,
pour son utilisation dans le traitement d'une maladie respiratoire inflammatoire ou obstructive, la composition étant administrée par inhalation.

2. Composition pour son utilisation selon la revendication 1, dans laquelle le β2-mimétique à longue durée d'action est le formotérol ou un solvate de celui-ci, ou le salmétérol ou des sels de celui-ci, ou des mélanges de ceux-ci.

3. Composition pour son utilisation selon l'une quelconque des revendications précédentes, adaptée pour une administration deux fois par jour ou une fois par jour.

4. Composition pour son utilisation selon l'une quelconque des revendications précédentes, la composition contenant en plus des composants (a) et (b), au moins un véhicule pharmaceutiquement acceptable.

5. Composition pour son utilisation selon la revendication 4, dans laquelle le véhicule a un diamètre maximal de particules allant jusqu'à 300 µm.

6. Composition pour son utilisation selon la revendication 4 ou 5, dans laquelle le véhicule est un saccharide.

7. Composition pour son utilisation selon la revendication 6, dans laquelle le saccharide est un monosaccharide, un disaccharide, un polysaccharide ou des alcools de sucre.

8. Composition pour son utilisation selon la revendication 7, dans laquelle le saccharide est l'arabinose, le glucose, le fructose, le ribose, le mannose, le saccharose, le tréhalose, le lactose, le maltose, des amidons, le dextrane ou le mannitol.

9. Composition pour son utilisation selon la revendication 8, dans laquelle le saccharide est le lactose.

10. Composition pour son utilisation selon l'une quelconque des revendications précédentes, la composition étant dans une forme appropriée pour l'inhalation par un dispositif inhalateur à monodose.

11. Composition pour son utilisation selon l'une quelconque des revendications précédentes, ladite composition étant dans une forme appropriée pour l'inhalation par un dispositif inhalateur à doses multiples.

12. Composition pour son utilisation selon l'une quelconque des revendications 1 à 11, **caractérisée en ce qu'**elle est contenue dans une capsule de gélatine dure ou d'hydroxypropylméthylcellulose.

13. Composition pour son utilisation selon l'une quelconque des revendications 1 à 11, **caractérisée en ce qu'**elle est contenue dans un blister.
